(19) Europäisches Patentamt
European Patent Office
Office européen des brevets

(11) Veröffentlichungsnummer: **0 318 468**
**A1**

(12) # EUROPÄISCHE PATENTANMELDUNG

(21) Anmeldenummer: **89100850.0**

(51) Int. Cl.4: **C 07 C 79/46**

(22) Anmeldetag: **05.05.86**

(30) Priorität: **15.05.85 DE 3517535**

(43) Veröffentlichungstag der Anmeldung:
**31.05.89 Patentblatt 89/22**

(84) Benannte Vertragsstaaten:
**AT BE CH DE FR GB IT LI NL SE**

(60) Veröffentlichungsnummer der früheren Anmeldung nach Art. 61 EPÜ: **0 201 829**

(71) Anmelder: **BAYER AG**
**D-5090 Leverkusen 1 Bayerwerk (DE)**

(72) Erfinder: **Grohe, Klaus, Dr.**
**Am Wasserturm 10**
**D-5090 Leverkusen 3 (DE)**

**Zeiler, Hans-Joachim, Dr.**
**Elsbeekerstrasse 46**
**D-5620 Velbert 15 (DE)**

**Metzger, Karl Georg, Dr.**
**Pahlkestrasse 75**
**D-5600 Wuppertal 1 (DE)**

(54) **2,4-Dichlor-5-fluor-3-nitro-benzoesäure und deren Derivate.**

(57) Die Erfindung betrifft neue Verbindungen der Formel (VII)

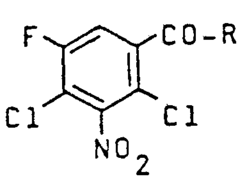

(VII)

in der R Hydroxyl, Halogen, insbesondere Chlor, oder Alkoxycarbonylmethyl mit 1 bis 4 Kohlenstoffatomen im Alkylteil bedeutet, und Verfahren zu deren Herstellung.

EP 0 318 468 A1

**Beschreibung**

**2,4-Dichlor-5-fluor-3-nitro-benzoesäure und deren Derivate**

Die Erfindung betrifft 2,4-Dichlor-5-fluor-3-nitro-benzoesäure, deren Derivate sowie Verfahren zu deren Herstellung.

Gegenstand der Erfindung sind Verbindungen der Formel (VII)

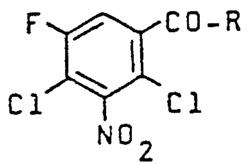

(VII)

in der
R Hydroxyl, Halogen, insbesondere Chlor, oder Alkoxycarbonylmethyl mit 1 - 5 Kohlenstoffatomen im Alkoxyteil bedeutet.

Ebenso ist Gegenstand der Erfindung ein Verfahren zur Herstellung von Verbindungen der Formel (VII)

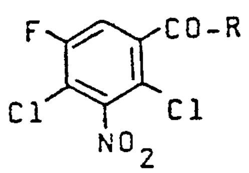

(VII)

in der
R Hydroxyl, Halogen, insbesondere Chlor oder Alkoxycarbonylmethyl mit 1 bis 4 Kohlenstoffatomen im Alkylteil bedeutet,
das dadurch gekennzeichnet ist, daß man 2,4-Dichlor-5-fluor-benzoesäure nitriert und die 2,4-Dichlor-5-fluor-3-nitro-benzoesäure gegebenenfalls in ein Säurehalogenid oder einen (2,4-Dichlor-5-fluor-3-nitro-benzoyl)-essigsäure-$C_1$-$C_4$-alkylester überführt.

Es wurde gefunden, daß die neuen 1-Aryl-4-chinolon-3-carbonsäuren der Formel (I),

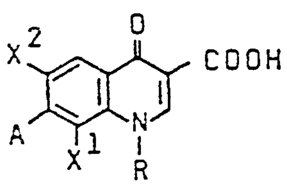

(I)

in welcher
$X^1$ für Nitro,
$X^2$ für Fluor steht,
R einen gegebenenfalls ein- oder mehrfach durch Halogenatome, Alkyl mit 1 - 4 Kohlenstoffatomen, Alkoxy, Alkylmercapto oder Alkylsulfonyl mit jeweils bis zu 3 Kohlenstoffatomen, Nitro, Cyano, Carboxyl, Methylendioxy sowie einen Aminrest

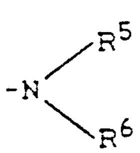

,

wobei $R^5$ und $R^6$ gleich oder verschieden sein können und Wasserstoff oder Alkyl mit 1 - 3 Kohlenstoffatomen bedeuten, substituierten Phenylrest oder einen aromatischen heterocyclischen Rest mit 5 - 7 Atomen bedeutet, wobei das Heteroatom S, O oder N sein kann und

A für 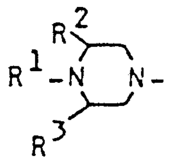 oder 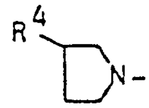 oder Halogen,

insbesondere Chlor oder Fluor steht, worin

$R^1$ für Wasserstoff, eine verzweigte oder unverzweigte Alkylgruppe mit 1 bis 4 Kohlenstoffatomen, die gegebenenfalls durch eine Hydroxy- oder Methoxygruppe substituiert sein kann, einen gegebenenfalls durch Hydroxy, Methoxy, Chlor oder Fluor substituierten Phenacylrest, einen Oxoalkylrest mit 2 bis 4 Kohlenstoffatomen, 4-Aminobenzyl, Formyl oder Acetyl,

oder für den Rest

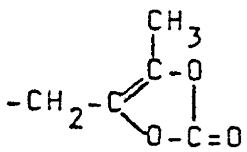 ,

$R^2$ für Wasserstoff, Methyl oder gegebenenfalls durch Chlor, Fluor, Methyl, Hydroxy oder Methoxy substituiertes Phenyl oder Thienyl,

$R^3$ für Wasserstoff oder Methyl und

$R^4$ für Wasserstoff, Hydroxy, Amino, Alkyl- oder Dialkylamino mit 1 oder 2 Kohlenstoffatomen in der Alkylgruppe, Hydroxymethyl, Aminomethyl, Alkyl- oder Dialkylaminomethyl mit 1 oder 2 Kohlenstoffatomen in der Alkylgruppe steht,

und deren pharmazeutisch verwendbare Hydrate, Säureadditionssalze, Alkali-, Erdalkali-, Silber- und Guanidiniumsalze, sowie in Form ihrer Ester und in anderen üblichen Prodrug-Formen eine hohe antibakterielle Wirkung aufweisen und sich ausgehend von Verbindungen der Formel (VII)

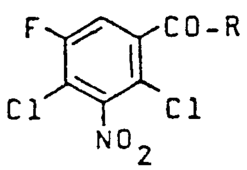

(VII)

in der

R Hydroxyl, Halogen, insbesondere Chlor, oder Alkoxycarbonylmethyl mit 1 bis 4 Kohlenstoffatomen im Alkylteil bedeutet,

herstellen lassen.

Die Endprodukte eignen sich als Wirkstoffe für die Human-und Veterinärmedizin, wobei zur Veterinärmedizin auch die Behandlung von Fischen zur Therapie oder Vorbeugung bakterieller Infektionen zu zählen ist.

Die Ausgangsprodukte der Formel VII, die Gegenstand der Erfindung sind, eignen sich bevorzugt zur Herstellung derjenigen Verbindungen der Formel (I), in denen

$X^1$ Nitro,

$X^2$ Fluor,

R für einen gegebenenfalls durch Halogen, Alkyl, Alkoxy, Alkylmercapto oder Alkylsulfonyl mit jeweils bis zu 2 Kohlenstoffatomen im Alkylteil, Nitro, Cyano substituierten Phenylrest oder einen Pyridin-, Thiophen-,Furan- oder Thiazolrest steht und

A für 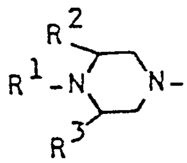 oder 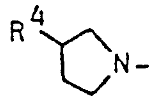 oder Halogen,

insbesondere Chlor oder Fluor steht, worin

$R^1$ für Wasserstoff, eine verzweigte oder unverzweigte Alkylgruppe mit 1 bis 3 Kohlenstoffatomen, die gegebenenfalls durch eine Hydroxygruppe substituiert sein kann, einen gegebenenfalls durch Chlor oder Fluor substituierten Phenacylrest, einen Oxoalkylrest mit 3 oder 4 Kohlenstoffatomen, 4-Aminobenzyl, Formyl oder Acetyl,

$R^2$ für Wasserstoff, Methyl oder gegebenenfalls durch Chlor oder Fluor substituiertes Phenyl,

$R^3$ für Wasserstoff oder Methyl und

$R^4$ für Wasserstoff, Hydroxy, Amino, Methylamino, Ethylamino, Aminomethyl, Methylaminomethyl, Ethylaminomethyl oder Diethylaminomethyl steht.

Außerdem eignen sich die Verbindungen der Formel (VII) zur Herstellung der Verbindungen der Formel (I), in denen

$X^1$ für Nitro,

$X^2$ für Fluro,

R für einen gegebenenfalls durch Chlor oder Fluor, Methyl, Methoxy, Methylmercapto, Nitro, Cyano substituierten Phenylrest steht, und

A für 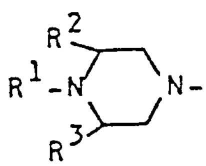 oder 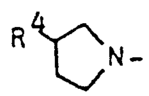 oder Halogen,

insbesondere Chlor oder Fluor steht, worin

$R^1$ für Wasserstoff, Methyl, Ethyl, 2-Hydroxyethyl, Phenacyl, 2-Oxopropyl, 3-Oxobutyl oder Formyl,

$R^2$ für Wasserstoff, Methyl oder Phenyl,

$R^3$ für Wasserstoff oder Methyl und

$R^4$ für Wasserstoff, Amino, Methylamino, Aminomethyl, Ethylaminomethyl oder Diethylaminomethyl steht.

Die Verbindungen der Formel (I) erhält man beispielsweise, wenn man die 7-Halogen-4-chinolon-3-carbonsäuren der Formel (II),

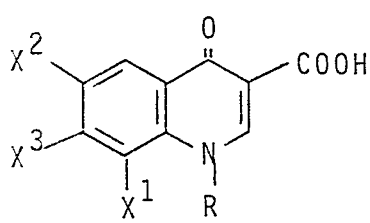

(II)

in welcher

R, $X^1$ und $X^2$ die oben angegebene Bedeutung haben und

$X^3$ für Chlor steht,

mit Aminen der Formel (III),

A - H (III)

in welcher

A die oben angegebene Bedeutung hat,

gegebenenfalls in Gegenwart von Säurebindern umsetzt.

Die als Ausgangsstoffe zur Herstellung von Verbindungen der Formel (I) verwendeten 7-Halogen-4-chinolon-3-carbonsäuren der Formel (II) können gemäß folgendem Reaktionsschema hergestellt werden:

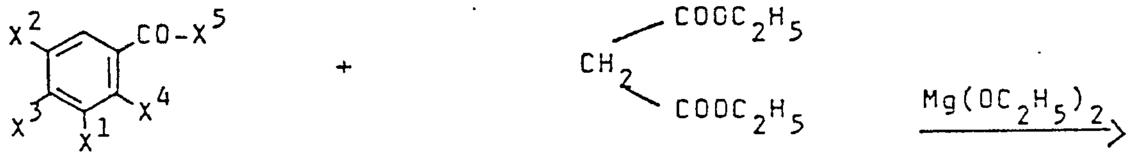


(1) (2)

$X^3$ = Cl

$X^4$ = Cl

$X^5$ = Cl, Br oder F

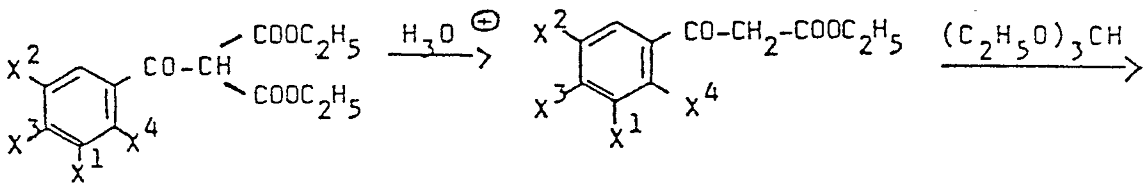


(3) (4)

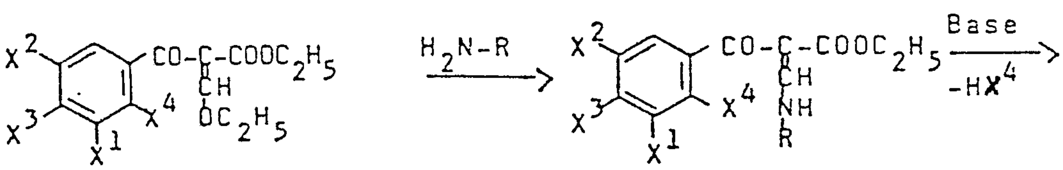


(5) (6)

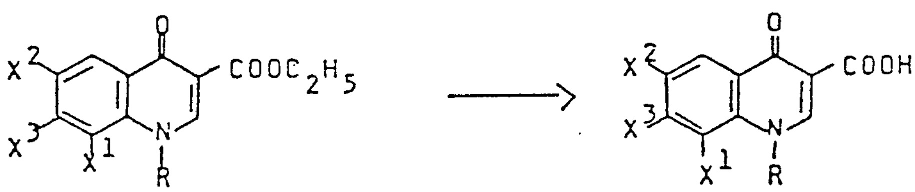

(7) (II)

Danach wird Malonsäurediethylester (2) in Gegenwart von Magnesiumethylat mit dem entsprechenden Benzoylhalogenid (1) zum Acylmalonester (3) acyliert (Organicum, 3. Auf. 1964. S. 438).

Durch partielle Verseifung und Decarboxylierung von (3) in wäßrigem Medium mit katalytischen Mengen Schwefelsäure oder 4-Toluolsulfonsäure erhält man in guter Ausbeute den Acylessigsäureethylester (4), der mit Orthoameisensäuretriethylester/Acetanhydrid in den 2-Benzoyl-3-ethoxy-acrylsäureethylester (5) übergeht. Die Umsetzung von (5) mit dem Amin $R\text{-}NH_2$ in einem Lösungsmittel, wie z.B. Methylenchlorid, einem Alkohol, Chloroform, Cyclohexan oder Toluol führt in leicht exothermer Reaktion zu den gewünschten Zwischenprodukten (6).

Die Cyclisierungsreaktion (6) → (7) wird in einem Temperaturbereich von eta 60 bis 300° C, bevorzugt 80 bis 180° C durchgeführt.

Als Verdünnungsmittel können Dioxan, Dimethylsulfoxid, N-Methylpyrrolidon, Sulfolan, Hexamethylphosphorsäuretrisamid und bevorzugt N,N-Dimethylformamid verwendet werden.

Als Säurebinder kommen für diese Reaktionsstufe Kaliumtert.-butanolat, Butyl-lithium, Lithium-phenyl, Phenylmagnesiumbromid, Natriummethylat, Natriumhydrid, Natrium-oder Kaliumcarbonat in Betracht. Besonders bevorzugt werden Kalium- oder Natriumfluorid, wenn Fluorwasserstoff abgespalten werden muß.

Es kann vorteilhaft sein, einen Überschuß von 10 Mol-% an Base einzusetzen.

Die in dem letzten Schritt erfolgende Esterhydrolyse von (7) unter basischen oder sauren Bedingungen führt zu den 4-Chinolon-3-carbonsäuren (II).

Das 2,4-Dichlor-5-fluor-3-nitro-benzoylchlorid wird durch Nitrierung der bekannten 2,4-Dichlor-5-fluor-benzoesäure zur 2,4-Dichlor-5-fluor-3-nitro-benzoesäure und deren Umsetzung mit Thionylchlorid erhalten.

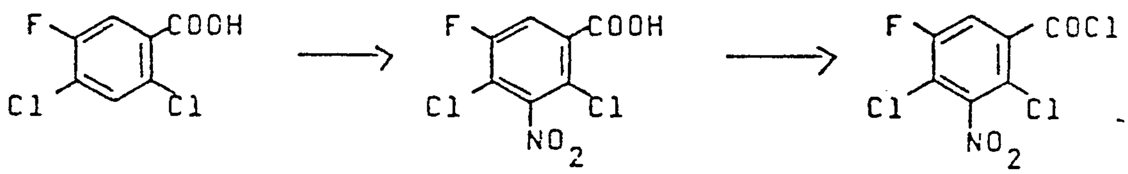

Beispiel A

7-Chlor-6-fluor-1,4-dihydro-4-oxo-8-nitro-1-phenyl-chinolin-3-carbonsäure

A) 2,4-Dichlor-5-fluor-3-nitro-benzoesäure

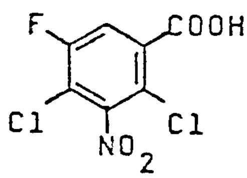

Unter Eiskühlung und Rühren werden 34 ml konz. Schwefelsäure tropfenweise mit 40 ml konz. Salpetersäure versetzt. In dieses Nitriergemisch trägt man portionsweise 20,9 g 2,4-Dichlor-5-fluorbenzoesäure ein, wobei die Temperatur auf 45 - 50° C steigt. Dann wird noch 3 Stunden auf 90 - 100° C erhitzt, das auf Raumtemperatur abgekühlte Gemisch auf 350 ml Eiswasser gegossen, der Niederschlag abgesaugt und mit Wasser gewaschen. Das feuchte Rohprodukt wird in 30 ml Methanol heiß gelöst und die Lösung mit 150 ml $H_2O$ versetzt.

Der Niederschlag wird kalt abgesaugt, mit $CH_3OH/H_2O$ gewaschen und i. Vak. bei 80° C getrocknet. Es werden 21,2 g rohe 2,4-Dichlor-5-fluor-3-nitro-benzoesäure erhalten. Sie ist genügend rein für die weiteren Umsetzungen. Eine Probe aus Toluol/Petrolether umkristallisiert liefert Kristalle vom Schmelzpunkt 192° C.

b) 2,4-Dichlor-5-fluor-3-nitro-benzoylchlorid

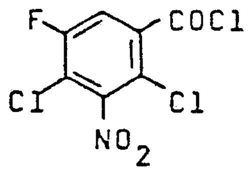

106,6 g 2,4-Dichlor-5-fluor-3-nitro-benzoesäure werden mit 250 ml Thionylchlorid 2 Stunden unter Rückfluß zum Sieden erhitzt. Das überschüssige Thionylchlorid wird dann bei Normaldruck abdestilliert und der Rückstand im Feinvak. fraktioniert. Bei 110 -115° C/0,08-0,09 mbar gehen 104,7 g 2,4-Dichlor-5-fluor-3-nitro-benzoylchlorid über. Beim Stehen bilden sich Kristalle vom Schmelzpunkt 35 - 37° C.

c) (2,4-Dichlor-5-fluor-3-nitro-benzoyl)-essigsäureethylester

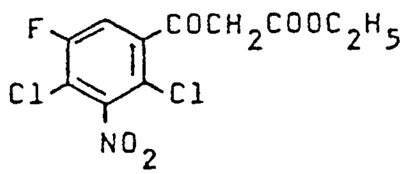

10,1 g Magnesiumspäne werden in 21 ml Ethanol mit 2.1 g Tetrachlormethan versetzt und nach Beginn der Wasserstoff-Entwicklung ein Gemisch aus 66,6 g Malonsäu rediethylester, 40 ml Ethanol und 150 ml Toluol bei 50 - 60° C tropfenweise zugefügt. Man rührt 1 Stunde bei dieser Temperatur nach, kühlt auf -5 bis -10° C und tropft langsam eine Lösung von 109,2 g 2,4-Dichlor-5-fluor-3-nitro-benzoylchlorid in 50 ml Toluol zu. Danach wird 1 Stunde bei 0° C gerührt, über Nacht auf Raumtemperatur gebracht und noch 2 Stunden auf 40 - 50° C erwärmt. Das Reaktionsgemisch wird unter Eiskühlung mit einem Gemisch aus 160 ml Wasser und 10,4 ml konzentrierter Schwefelsäure versetzt und die organische Phase abgetrennt. Die wäßrige Phase wird mit Toluol extrahiert und der vereinigte organische Extrakt mit gesättigter Natriumchlorid-Lösung gewaschen, mit Natriumsulfat getrocknet und das Lösungsmittel abgezogen. Man erhält 144,5 g (2,4-Dichlor-5-fluor-3-nitro-benzoyl)-malonsäurediethylester als Rohprodukt. Dieses wird nach Zugabe von 200 ml Wasser und 0,6 g 4-Toluolsulfonsäure 3 Stunden unter Rückfluß erhitzt, die Mischung mit Methylenchlorid extrahiert, der Extrakt mit Natriumsulfat getrocknet und das Lösungsmittel im Vakuum abdestilliert. Es werden 118 g substituierter Benzoylessigester als Rohprodukt erhalten. Er besitzt eine für die weiteren Umsetzungen genügende Reinheit.

d) 7-Chlor-6-fluor-1,4-dihydro-4-oxo-8-nitro-1-phenyl-chinolin-3-carbonsäure

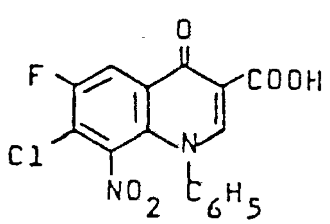

244,8 g (2,4-Dichlor-5-fluor-3-nitro-benzoyl)-essigsäureethylester werden mit 166 g Orthoameisensäuretriethylester und 185 g Essigsäureanhydrid 3 Stunden auf 150 -1 60° C erhitzt. Man engt im Vak. ein und erhält 270 g 2-(2,4-Dichlor-5-fluor-3-nitro-benzoyl)-3-ethoxy-acrylsäure-ethylester als öligen Rückstand.

38 g dieser Zwischenstufe werden in 100 ml Ethanol unter Eiskühlung tropfenweise mit 9,5 g Anilin versetzt und 1 Stunde bei 20° gerührt. Das ausgefallene Produkt wird nach Zugabe von 100 ml Wasser abgesaugt, mit Ethanol/$H_2O$ (1:1) gewaschen und getrocknet. Man erhält 37,9 g 2-(2,4- Dichlor-5-fluor-3-nitro-benzoyl)-3-anilino-acrylsäure ethylester (6) ($R = C_6H_5$, $X^1 = NO_2$, $X^2 = F$, $X^3 = X^4 = Cl$) vom Schmelzpunkt 133 - 135° C.

37 g der vorgenannten Verbindung werden in 100 ml wasserfreiem Dioxan mit 13,3 g DBU versetzt und 4

Std. auf 100°C erhitzt. Das Lösungsmittel wird im Vakuum abdestilliert, der Rückstand in Methylenchlorid/ Wasser aufgenommen, die Methylenchlorid-Phase abgetrennt, mit Natriumsulfat getrocknet und das Methylenchlorid abdestilliert. Es werden 32 g 7-Chlor-6-fluor-1,4-dihydro-4-oxo-8-nitro-1-phenyl-chinolin-3-carbonsäureethylester als Rohprodukt erhalten. Nach Umkristallisation aus Ethanol schmelzen die hellbraunen Kristalle bei 186 - 188°C.

10,5 g dieses Esters werden in einem Gemisch aus 100 ml Essigsäure, 70ml Wasser und 10ml konzentrierter Schwefelsäure 2 Std. auf 150°C erhitzt. Die Suspension wird in 300 ml Eiswasser gegossen, der Niederschlag abgesaugt, mit Wasser und Methanol gewaschen und im Vakuum getrocknet.
Ausbeute: 7,5 g 7-Chlor-6-fluor-1,4-dihydro-4-oxo-8-nitro- 1-phenyl-chinolin-3-carbonsäure vom Schmelzpunkt 249 - 250°C.

Beispiel 1 (nicht Gegenstand der Erfindung)

6,8-Difluor-1,4-dihydro-4-oxo-1-(4-fluorphenyl)-7-(4-methyl-1-piperazinyl)-chinolin-3-carbonsäure

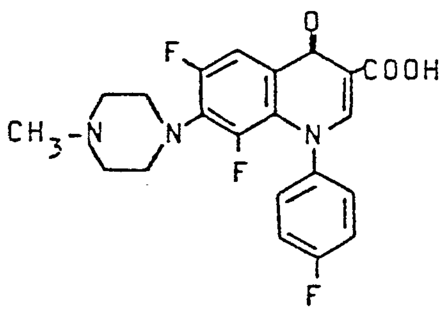

3 g 6,7,8-Trifluor-1,4-dihydro-4-oxo-1-(4-fluor-phenyl)-chinolin-3-carbonsäure werden in 30 ml Pyridin mit 2,7 g N-Methylpiperazin 6 Std. refluxiert. Das Lösungsmittel wird im Vak. abdestilliert, der Rückstand in 30 ml Wasser aufgenommen, der Niederschlag kalt abgesaugt, mit Wasser gewaschen, im Vak. über Calciumchlorid bei 100°C getrocknet und aus Glykolmonomethylether umkristallisiert.
Ausbeute: 2,5 g. Schmelzpunkt: 274 - 277°C (unter Zersetzung).
Mit konz. Salzsäure erhält man analog Beispiel 3 das Hydrochlorid.
Schmelzpunkt: >300°C (unter Zersetzung).

Beispiel 2

6-Fluor-1,4-dihydro-4-oxo-8-nitro-1-phenyl-7-(4-methyl-1-piperazinyl)-chinolin-3-carbonsäure

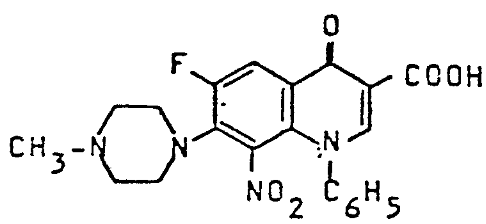

Die Synthese erfolgt analog Beispiel 1 (2 Std. Rückfluß). Als Ausgangsprodukte werden die 7-Chlor-6-fluor-1,4-dihydro-4-oxo-8-nitro-1-phenyl-chinolin-3-carbonsäure und N-Methyl-piperazine verwendet. Das als Reaktionsprodukt erhaltene Chinolin-3-carbonsäure-Hydrochlorid (I) ($R = C_6H_5$, $X^1 = NO_2$, $X^2 = F$, A = 4-Methyl-1-piperazinyl) besitzt einen Schmelzpunkt >300°C (unter Zersetzung). Schmelzpunkt des Betains : 240-245°C.

Die Verbindung besitzt ausgezeichnete antibakterielle Eigenschaften.

**Patentansprüche**

1. Verbindungen der Formel (VII)

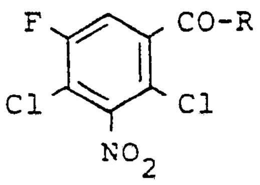

in der
R Hydroxyl, Halogen, insbesondere Chlor, oder Alkoxycarbonylmethyl mit 1 bis 4 Kohlenstoffatomen im Alkylteil bedeutet.

2. Verfahren zur Herstellung von Verbindungen der Formel (VII)

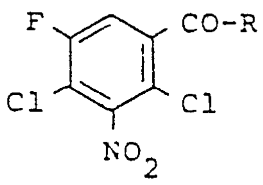

in der
R Hydroxyl, Halogen, insbesondere Chlor, oder Alkoxycarbonylmethyl mit 1 bis 4 Kohlenstoffatomen im Alkylteil bedeutet,
dadurch gekennzeichnet, daß man 2,4-Dichlor-5-fluorbenzoesäure nitriert und die 2,4-Dichlor-5-fluor-3-nitro-benzoesäure gegebenenfalls in ein Säurehalogenid oder einen (2,4-Dichlor-5-fluor-3-nitro-benzoyl)-essigsäure-$C_1$-$C_4$-alkylester überführt.

Europäisches Patentamt

# EUROPÄISCHER RECHERCHENBERICHT

Nummer der Anmeldung

EP 89 10 0850

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CHEMICAL ABSTRACTS, Band 85, Nr. 11, 13. September 1976, Seite 524, Zusammenfassung 77903j, Columbus, Ohio, US; & HU-A-10 919 (J. SERES et al.) 29-12-1975<br>----- | 1 | C 07 C 79/46 |
| | | | **RECHERCHIERTE SACHGEBIETE (Int. Cl.4)** |
| | | | C 07 C 79/00 |

Der vorliegende Recherchenbericht wurde für alle Patentansprüche erstellt

| Recherchenort | Abschlußdatum der Recherche | Prüfer |
|---|---|---|
| DEN HAAG | 01-03-1989 | KLAG M.J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)